# EUROPEAN PATENT APPLICATION

(11) **EP 4 026 911 A1**
(43) Date of publication of application: **13.07.2022**
(21) Application number: 20861179.8
(22) Date of filing: 04.09.2020
(51) Int. Cl.: C12Q 1/37

(54) **TRYPTASE ACTIVITY MEASUREMENT SUBSTRATE**

(30) Priority: 05.09.2019 JP 2019161824
(71) Applicant: National University Corporation Tokyo University Of Agriculture and Technology, Fuchu-shi Tokyo 183-8538 (JP); Peptide Support Co. Ltd., Kitayushi-shi, Fukuoka 808-0135 (JP)
(72) Inventor: MATSUDA, Hiroshi, Fuchu-shi, Tokyo 183-8538 (JP); NISHINO, Norikazu, Kitakyushu-shi, Fukuoka 808-0104 (JP)
(74) Representative: D Young & Co LLP
(86) International application number: PCT/JP2020/033556
(87) International publication number: WO 2021/045182

(57) **Abstract**

An object of the present invention is to provide a method for measuring tryptase activity in a blood sample accurately and rapidly by a convenient operation in order to accurately evaluate the state of a disease whose state involves mast cells. The present invention enables tryptase activity in a blood sample to be directly measured without the pretreatment, such as purification or concentration, of the blood sample, using a substrate for measuring tryptase activity, comprising a tripeptide C-terminally linked through a peptide bond to a dye label, selected from the following formulas (1) to (3): (1) Lys-Ala-Arg-X, (2) Ala-Ala-Arg-X, and (3) Abu-Ala-Arg-X (wherein X represents a dye label whose fluorescence characteristics or color development characteristics change upon the cleavage of the peptide bond with Arg, and Abu represents 2-aminobutyric acid) .

## Description

### Technical Field

The present invention relates to a substrate for measuring tryptase activity, comprising a predetermined tripeptide C-terminally linked through a peptide bond to a dye label, wherein fluorescence characteristics or color development characteristics of the dye label change upon the cleavage of the C-terminal peptide bond of the tripeptide, a method for measuring tryptase activity in a blood sample using the substrate, and a kit for measuring tryptase activity in a blood sample, comprising the substrate.

### Background Art

Diseases whose state particularly involves mast cells, such as allergy disease, tumor, or retinopathy of prematurity, are diagnosed as mast cell activation syndrome. Among a wide variety of mediators that are released from mast cells, a proteolytic enzyme tryptase is used in disease state evaluation because of its specificity. Immune reaction (ELISA) utilizing a specific antibody is currently used worldwide for the quantification thereof. However, ELISA, which can merely determine the abundance of an antigenic tryptase-related protein, is insufficient because tryptase functions as an enzyme, affecting living bodies and therefore, the measurement of enzyme activity is important for the assessment of the disease state.

The present inventors have reported that tryptase released from mast cells is essential for the onset of retinopathy of prematurity (non-patent document 1). In this report, the present inventors have determined the involvement of tryptase using ELISA. As mentioned above, the measurement of enzyme activity is essential if one consider how the disease states (whether to become blind or selection of a treatment method) of newborns could be evaluated.

Tryptase is an enzyme that is released into blood by mast cell degranulation, and its concentration in blood is often low, on the order of 10 µg/L (non-patent documents 2 and 3; non-patent document 3 has reported that the abundance in the serum of healthy infants under the age of 1 is 4.67 µg/L). Since tryptase having this concentration cannot be detected using a conventional tryptase substrate, it is necessary to concentrate a specimen solution or to lengthen a reaction time. As a result, accurate and rapid measurement is difficult under these circumstances. For convenient measurement of tryptase activity, it is desirable to subject serum which facilitates sample preparation to direct measurement. However, serum contains thrombin, a protease of a blood coagulation system. In the case of measuring tryptase activity using a protease substrate known in the art, thrombin exhibits much larger activity than that of tryptase and thus interferes with the measurement.

In enzymatic chemical research on tryptase from the 1980 onward, Boc-Phe-Ser-Arg-MCA, Tos-Gly-Pro-Arg-MCA, or the like, which has been commercially available as a fluorescent substrate of trypsin from back then, has been used. In addition, the substrate specificity of several types of tripeptide-MCA has been examined, though substrates other than the two types described above are not generally used (non-patent documents 4 to 16). Since tryptase is a trypsin-like enzyme, its enzyme activity can be detected using any MCA substrate having C-terminal Arg, whether the responsiveness (susceptibility) is high or low. Therefore, the optimization of the amino acid sequences of the tripeptides has not yet been performed.

In recent years, Ac-Lys-Pro-Arg-FMCA (7-amino-4-trifluoromethylcoumarin) has been reported as a good substrate for tryptase (non-patent document 17). The presence of Lys in this sequence provides a valuable insight as to the sites that act on natural substrate proteins for tryptase. Also, comprehensive library construction to examine the substrate specificity of protease was performed as early as 20 years ago by an approach of constructing tripeptide libraries on base plates, and -Lys/Arg-Asn-Lys/Arg- was found as a tripeptide sequence having the highest responsiveness to beta tryptase I and II (non-patent document 18). Asn at position P₂ exhibited slightly more than two times the responsiveness of Ala at position P₂, and further, at position P₃, an amino acid having a positively charged side chain had sensitivity about two times better than that of Ala at position P₃. Unfortunately, this excellent research outcome published in 2001 was not put in practical use in an easy-to-use form such as ⁱBoc-Arg-Asn-Arg-MCA.

As described above, reports on tripeptides capable of serving as substrates for tryptase have previously been made. However, to inventors' knowledge, there is no report on the use of these substrates in the direct measurement of blood samples contaminated with proteases. Furthermore, the substrates may be cleaved by other proteases such as thrombin. Therefore, technical difficulty still remains in the synthesis of a substrate for measuring tryptase activity that allows even a blood sample contaminated with proteases to be directly measured.

### Prior Art Documents

### Non-patent Documents

Non-patent document 1: J Clin Invest., 127 (11), 3987-4000, 2017
Non-patent document 2: Thermo Fisher Scientific Inc., "Mast cell Tryptase For diagnosis and prediction" (http://www.phadia.com/Global/Market%20Companies/Finland/M ast%20Cell%20Tryptase%20for%20Diagnosis%20and%20Prediction .pdf)
Non-patent document 3: Allergy Asthma Proc. 35, 404-408, 2014
Non-patent document 4: J. Biol. Chem., 258, 13552-13557, 1983
Non-patent document 5: J. Biol. Chem., 262, 1363-1373, 1987
Non-patent document 6: Biol. Chem. Hoppe-Seyler, 369, 617-625, 1988
Non-patent document 7: J. Biol. Chem., 263, 18104-18107, 1988
Non-patent document 8: Biol. Chem. Hoppe-Seyler Suppl., 371,65-73, 1990
Non-patent document 9: J. Biol. Chem., 267, 13573-13579, 1992
Non-patent document 10: J. Biochem., 120, 856-864, 1996
Non-patent document 11: Peptides, 19, 437-443, 1998
Non-patent document 12: FEBS Lett., 408, 85-88, 1997
Non-patent document 13: British J. Pharm., 138, 959-967, 2003
Non-patent document 14: Allergology International, 57, 83-91, 2008
Non-patent document 15: Chemical Science, 6, 1792-1800, 2015
Non-patent document 16: IJC Metabolic & Endocrine, 10, 16-23, 2016
Non-patent document 17: Sigma-Aldrich Co. LLC, "N-Acetyl-Lys-Pro-Arg-7-Amino-4-Trifluoromethylcoumarin" (product number: C6608), product information (https://www.sigmaaldrich.com/content/dam/sigma-aldrich/docs/Sigma/Datasheet/3/c6608dat.pdf)
Non-patent document 18: J. Biol. Chem., 276, 34941-34947, 2001

### Summary of the Invention

### Object to be Solved by the Invention

An object of the present invention is to provide a method for measuring tryptase activity in a blood sample accurately and rapidly by a convenient operation in order to accurately evaluate the state of a disease whose state involves mast cells.

### Means to Solve the Object

As mentioned above, in enzymatic chemical research on tryptase, Boc-Phe-Ser-Arg-MCA, Tos-Gly-Pro-Arg-MCA, or the like, which has been commercially available as a fluorescent substrate of trypsin from back then, has been used. These substrates exhibit sufficient activity for measuring the activity of purified tryptase. However, the present inventors have recognized, as problems, that these substrates have low responsiveness from the viewpoint of measuring tryptase activity in serum and cannot be used in the activity measurement of tryptase in serum because of being interfered with by thrombin, as mentioned above. The present inventors have conducted diligent studies to solve these problems and consequently found that ⁱBoc-Ala-Ala-Arg-MCA, ⁱBoc-Lys-Ala-Arg-MCA, ⁱBoc-Abu-Ala-Arg-MCA, and ⁱBoc-Lys-Pro-Arg-MCA have very high responsiveness to tryptase and their cleavage are detectable even by tryptase having a low concentration. The present inventors have further found that a macromolecular substrate prepared by linking the tripeptide-MCA to poly(L-lysine) via succinic acid allows tryptase activity in serum to be specifically measured, without being affected by the action of thrombin in a complex, even in the presence of thrombin after reaction with α2 macroglobulin. The present invention has been completed on the basis of these findings.

Specifically, the present invention is specified by the following items.
[1] A substrate for measuring tryptase activity, comprising a tripeptide C-terminally linked through a peptide bond to a dye label (tripeptide-X), selected from the following formulas (1) to (3):
   (1)

      Lys-Ala-Arg-X,
   (2)

      Ala-Ala-Arg-X,

      and
   (3)

      Abu-Ala-Arg-X
   (wherein X represents a dye label whose fluorescence characteristics or color development characteristics change upon the cleavage of the peptide bond with Arg, and Abu represents 2-aminobutyric acid).
[2] The substrate for measuring tryptase activity according to [1], wherein the substrate for measuring tryptase activity is for directly measuring tryptase activity in a blood sample.
[3] The substrate for measuring tryptase activity according to [1] or [2], wherein the tripeptide-X is N-terminally linked to a poorly tryptase-digestible water-soluble polymer having a molecular weight of 5,000 or higher.
[4] The substrate for measuring tryptase activity according to [3], wherein the poorly tryptase-digestible water-soluble polymer is a polyamino acid.
[5] The substrate for measuring tryptase activity according to [4], wherein the polyamino acid is selected from poly(L-lysine), dendritic poly(L-lysine), poly(D-lysine), dendritic poly(D-lysine), poly(L-ornithine) and poly(D-ornithine).
[6] The substrate for measuring tryptase activity according to any one of [1] to [5], wherein the dye label is a fluorescent dye label selected from an MCA group, an ANS group, a CMCA group, an FMCA group, an AMP group, a rhodamine 110 group, a rhodamine 110 monoamide group, a rhodamine 6G group and a rhodamine B group.
[7] A method for measuring tryptase activity in a blood sample, comprising the following steps (A) and (B):
   (A) contacting the substrate for measuring tryptase activity according to any one of [1] to [6] with the blood sample; and
   (B) calculating a degree of the tryptase activity in the blood sample by measuring an amount of change in fluorescence characteristics or color development characteristics of a dye label after the step (A).
[8] The method according to [7], wherein the blood sample is serum.
[9] A kit for measuring tryptase activity in a blood sample, comprising the substrate for measuring tryptase activity according to any one of [1] to [6].
[10] The kit according to [9], wherein the blood sample is serum.

### Effect of the Invention

The present invention enables tryptase activity in a blood sample to be directly measured without the pretreatment, such as purification or concentration, of the blood sample.

### Mode of Carrying Out the Invention

The present invention relates to a substrate for measuring tryptase activity, comprising a tripeptide C-terminally linked through a peptide bond to a dye label (tripeptide-X), selected from the following formulas (1) to (3) (hereinafter, also referred to as the "substrate of the present invention"), a method for measuring tryptase activity in a blood sample using the substrate of the present invention (hereinafter, also referred to as the "measurement method of the present invention"), and a kit for measuring tryptase activity in a blood sample, comprising the substrate of the present invention (hereinafter, also referred to as the "kit of the present invention").
(1)

   Lys-Ala-Arg-X,
(2)

   Ala-Ala-Arg-X,

   and
(3)

   Abu-Ala-Arg-X
(wherein X represents a dye label whose fluorescence characteristics or color development characteristics change upon the cleavage of the peptide bond with Arg, and Abu represents 2-aminobutyric acid).

The substrate of the present invention can be suitably used for the purpose of directly measuring tryptase activity in a blood sample.

As used herein, the term "cleavage" in relation to peptides is used interchangeably with "hydrolysis". The substrate of the present invention allows tryptase activity to be detected even at a tryptase concentration as very low as approximately 10 µg/L. Therefore, even tryptase in a blood sample can be evaluated for its activity by a method of, for example, waiting for enzyme reaction to become a steady state for 2 minutes after the start of measurement and then recording, as enzyme activity, increase in fluorescence intensity obtained for 1 minute from 2 minutes to 3 minutes after the start of measurement.

The dye label is not limited as long as fluorescence characteristics or color development characteristics of the dye label change upon the cleavage of the peptide bond. A fluorescent label is preferred from the viewpoint of convenient detection and sensitivity. Examples thereof can include an MCA (4-methyl-coumaryl-7-amide) group, an ANS (2-aminonaphthalene-6-sulfonic acid) group, a CMCA (7-amino-4-chloromethylcoumarin) group, an FMCA (7-amino-4-trifluoromethylcoumarin) group, an AMP (2-amino-7-methylpurine-6-thiol) group, a rhodamine 110 group, a rhodamine 110 monoamide group, a rhodamine 6G group, and a rhodamine B group.

The presence or absence of change in the fluorescence characteristics or the amount of the change can be detected or quantified using a fluorescence intensity measuring machine or the like known in the art. The presence or absence of change in the color development characteristics or the amount of the change can be detected or quantified using a spectrophotometer measuring machine or the like. In this respect, it is preferred to select an excitation wavelength and a fluorescence wavelength such that the dye label can be analyzed for its absorption wavelength or fluorescence wavelength and specifically detected or quantified. In the case of using an MCA group as the dye label in the substrate of the present invention, it is preferred for 1) specifically measuring AMC (7-amino-4-methylcoumarin) to detect or measure a fluorescence wavelength (e.g., 400 nm to 500 nm) emitted from AMC by irradiation with a wavelength that excites AMC without exciting the MCA group (e.g., 360 nm to 400 nm), and it is preferred for 2) specifically measuring the MCA group to detect or measure a wavelength (e.g., 350 nm to 385 nm) emitted from the MCA group by irradiation with a wavelength that excites the MCA group without exciting AMC (e.g., 260 nm to 350 nm).

In the present specification, the blood sample can be a blood sample collected from a test subject. Examples thereof can include whole blood, plasma, and serum. Plasma or serum is preferred. Serum is more preferred because of convenient measurement and easy sample preparation. The blood sample may optionally be supplemented with a blood coagulation inhibitor. The substrate for measuring tryptase activity of the present invention comprises a recognition sequence by thrombin present in blood. Therefore, the blood sample may contain a thrombin inhibitor such as antithrombin III, hirudin, heparin cofactor II, or aprotinin in order to prevent interference by thrombin. In a preferred aspect, the blood sample contains no thrombin inhibitor. Examples of the test subject can preferably include a mammal, more preferably a primate, further preferably a human.

In the substrate of the present invention, the tripeptide may be linked to a poorly tryptase-digestible water-soluble polymer having a weight-average molecular weight of 5,000 or higher, more preferably 6,000 or higher, further preferably 7,000 or higher, still further preferably 8,000 or higher. In this aspect, the substrate of the present invention does not undergo cleavage by thrombin that coexists in the blood sample, and therefore allows tryptase activity to be precisely measured even without the pretreatment, such as the addition of a thrombin inhibitor or the removal of thrombin, of the blood sample. This is presumably because a substrate for measuring tryptase activity having a weight-average molecular weight of 5,000 or higher does not reach the active center of thrombin captured by α2 macroglobulin tetramer in the blood sample. In this aspect, the tripeptide selected from the formulas (1) to (3) as well as a known tripeptide (Lys-Pro-Arg, a tripeptide selected from the formulas (1) to (3), wherein position P₂ is Asn, Lys/Arg-Asn-Lys/Arg, etc.) having responsiveness to tryptase equivalent to or higher than that of the tripeptide selected from the formulas (1) to (3) can be used as the tripeptide.

The poorly tryptase-digestible water-soluble polymer can be a polymer that does not hinder the cleavage, by tryptase, of the peptide bond between the Arg residue and the dye label in the substrate of the present invention. Examples of the upper limit of the weight-average molecular weight can include 5,000,000 or lower, 1,000,000 or lower, 500,000 or lower, 100,000 or lower, and 50,000 or lower. The term "poorly tryptase-digestible" means that in the substrate of the present invention, a bond in the poorly tryptase-digestible water-soluble polymer is more difficult to cleave by tryptase as compared with the peptide bond between the Arg residue and the dye label, and means that, for example, the responsiveness of the poorly tryptase-digestible water-soluble polymer to tryptase is 1/10 or less, preferably 1/50 or less, more preferably 1/100 or less, further preferably 1/500 or less, still further preferably 1/1000 or less, of the responsiveness of the peptide bond between the Arg residue and the dye label to tryptase. In this context, high responsiveness to tryptase is not desirable because the water-soluble polymer is digested, during reaction, into a small molecular size that allows the substrate to reach the active center of thrombin captured by α2 macroglobulin tetramer. Although not bound by any theory, the essential nature of the poorly tryptase-digestible water-soluble polymer is too large a molecular weight to deliver the supported tripeptide-MCA substrate to the active center of an interfering enzyme such as thrombin trapped by α2 macroglobulin tetramer. Tryptase has a molecular weight of 31 to 33 kDa and functions as a tetramer (135 kDa) and is therefore considered not to be inhibited by α2 macroglobulin.

The poorly tryptase-digestible water-soluble polymer is preferably a polyamino acid. Particularly, preferred examples thereof can include poly(L-lysine), dendritic poly(L-lysine), poly(D-lysine), dendritic poly(D-lysine), poly(L-ornithine) and poly(D-ornithine). The poorly tryptase-digestible water-soluble polymer is preferably linked to the N terminus of the tripeptide and may be linked to the tripeptide via a linker such as PEG (polyethylene glycol) or succinic acid.

The substrate of the present invention can be suitably used in a method for measuring tryptase activity in a blood sample, comprising in order the steps of: (A) contacting the substrate of the present invention with the blood sample; and (B) calculating a degree of the tryptase activity in the blood sample by measuring an amount of change in fluorescence characteristics or color development characteristics of the dye label after the step (A) (the measurement method of the present invention). The contact time and the amount of the substrate added to the blood sample in the step (A) can be appropriately set depending on the tryptase activity in the blood sample. Examples of the method for calculating the degree of the tryptase activity in the blood sample in the step (B) can include a method of calculating the tryptase activity in the blood sample on the basis of a calibration curve created in advance using purified tryptase and the substrate of the present invention. In the measurement method of the present invention, the thrombin inhibitor may be added to the blood sample to be measured prior to the step (A) in order to prevent interference by thrombin. When the substrate of the present invention is in a form linked to a poorly tryptase-digestible water-soluble polymer having a molecular weight of 5,000 or higher, the thrombin inhibitor may not be added. Tryptase activity, as criteria for determining a disease, in the blood sample may be appropriately set according to the disease. Since a tryptase concentration of 10 µg/L or higher in blood is generally considered as a high level, criteria for determining a pathological condition may be set on the basis of activity at a tryptase concentration of 10 µg/L.

In one aspect, the substrate of the present invention may be preserved, distributed and used as a kit for measuring tryptase activity in a blood sample (the kit of the present invention). The kit of the present invention is used in the measurement method of the present invention. The kit of the present invention may comprise a container for blood sample collection, a pretreatment agent for the blood sample, a reaction buffer, a preservative, an instruction manual, and the like, in addition to the substrate of the present invention. Examples of the pretreatment agent for the blood sample can include the thrombin inhibitor and the blood coagulation inhibitor. In the case of using the substrate of the present invention in a form linked to a poorly tryptase-digestible water-soluble polymer having a molecular weight of 5,000 or higher, the kit may not comprise the thrombin inhibitor.

Use of the substrate, the measurement method or the kit of the present invention enables the disease state of mast cell activation syndrome to be evaluated. In this context, examples of the mast cell activation syndrome can include allergy, atopic dermatitis, tumor, retinopathy of prematurity, systemic mast cell disease, and chronic lymphatic leukemia (CLL). According to the present invention, not only the abundance of a tryptase protein-related antigen but also tryptase activity in blood can be directly measured. Therefore, a disease state (stage, etc.) can be accurately evaluated as compared with a conventional method using ELISA.

Hereinafter, the present invention will be described more specifically with reference to Examples. However, the technical scope of the present invention is not limited by these examples.

### Example 1

### 1. Screening of tryptase substrate

A total of 17 types of MCA substrates were chemically synthesized by adopting document information on the amino acid sequences of previously reported tripeptide-MCA substrates used for tryptase, and a simplified library approach. ⁱBoc-Ala-Ala-Arg-MCA having the best responsiveness and physical properties was found for commercially available tryptase separated from the human lung. ⁱBoc-Ala-Ala-Arg-MCA_Pmcs was synthesized by the following procedures.
(1) Under ice cooling, N,N'-dicyclohexylcarbodiimide (DCC) (450 mg, 2.2 mmol) was added to a solution of Fmoc-Arg(Pmc)-OH (manufactured by Watanabe Chemical Industries, Ltd., 4 mmol) in dichloromethane (DCM), and the mixture was stirred for 1 hour. AMC (manufactured by Tokyo Chemical Industry Co., Ltd., 400 mg, 2.2 mmol) was added to the formed symmetric acid anhydride, and the mixture was reacted overnight at room temperature to obtain Fmoc-Arg(Pmc)-MCA.
(2) ⁱBoc-Ala-OH (own made, 1.89 g, 10 mmol) and H-Ala-OBzl_HCl (own made, 2.12 g, 10 mmol) were condensed according to a standard method to prepare ⁱBoc-Ala-Ala-OBzl. Further, catalytic reduction was performed in methanol using 5% Pd-C to obtain ⁱBoc-Ala-Ala-OH [yield: 2.08 g (80%)].
(3) Fmoc-Arg(Pmc)-MCA (1.0 mmol) was dissolved in 20% piperidine (5 mL), and the solution was reacted at room temperature for 2 hours. The solvent was distilled off, and the residue was dissolved in N,N'-dimethylformamide (DMF). To this solution, ⁱBoc-Ala-Ala-OH (260 mg, 1.0 mmol) was added, followed by condensation by the HBTU/HOBt method. The product ⁱBoc-Ala-Ala-Arg(Pmc)-MCA was purified by silica gel chromatography to obtain 420 mg (50%) of white crystals.
(4) ⁱBoc-Ala-Ala-Arg(Pmc)-MCA (400 mg, 0.45 mmol) was dissolved in trifluoroacetic acid (TFA) (5 mL), and the solution was reacted at room temperature for 2 hours. TFA was distilled off, and diethyl ether was added to the residue to obtain Pmcs salt as a crystalline powder [yield: 365 mg (91%)].

In addition to reference to past documents as described above, ⁱBoc-Y-X-Arg-MCA in which Gly, Ala, Abu, Val, Pro, Phe or hydrophilic Lys having distinctive bulkiness was introduced to positions P₂ and P₃ was synthesized as listed in Table 1 by a peptide library approach, and activity measurement was performed. Abu (2-aminobutyric acid) is a non-protein amino acid, but was added as an optimum peptide sequence search tool.

The activity measurement was performed by the following method.
(1) A stock of a substrate solution was provided as a 2.0 mM solution in DMSO.
(2) The stock of the substrate solution was diluted 20-fold with 50 mM Tris-HCl (pH 8.0) to prepare a 100 µM solution. 100 µL thereof was added to a polypropylene microtube, further 2 µL of a commercially available tryptase solution (manufactured by Cappel Laboratories, Inc.) was added thereto, and the tube was lightly vortexed for mixing. Immediately thereafter, change in fluorescence intensity at 470 nm was measured at an excitation wavelength of 365 nm using an enzyme activity measurement apparatus (manufactured by Peptide Support Ltd., prototype).

**[Table 1]**

| | | |
|---|---|---|
| Amino acid sequence of fluorescent substrate and comparison of responsiveness to human lung tryptase*¹ | | |

| | *ⁱ*Boc-P₃-P₂-Arg-MCA*² | Tryptase activity*³ |
|---|---|---|
| | *ⁱ*Boc-Phe-Ser-Arg-MCA | 11 |
| | *ⁱ*Boc-Arg-MCA | 0 |
| | *ⁱ*Boc-Gly-Gly-Arg-MCA | 0 |
| | *ⁱ*Boc-Phe-Gly-Arg-MCA | 5 |
| | *ⁱ*Boc-Lys-Gly-Arg-MCA | 75 |
| | *ⁱ*Boc-Ala-Ala-Arg-MCA | 100 |
| | *ⁱ*Boc-Abu-Ala-Arg-MCA | 109 |
| | *ⁱ*Boc-Val-Ala-Arg-MCA | 53 |
| | *ⁱ*Boc-Phe-Ala-Arg-MCA | 62 |
| | *ⁱ*Boc-Lys-Ala-Arg-MCA | 167 |
| | *ⁱ*Boc-Ala-Abu-Arg-MCA | 80 |
| | *ⁱ*Boc-Abu-Abu-Arg-MCA | 69 |
| | *ⁱ*Boc-Gly-Pro-Arg-MCA | 9 |
| | Tos-Gly-Pro-Arg-MCA | 25 |
| | *ⁱ*Boc-Val-Pro-Arg-MCA | 20 |
| | *ⁱ*Boc-Lys-Pro-Arg-MCA | 175 |
| | *ⁱ*Boc-Ala-Val-Arg-MCA | 50 |
| | *ⁱ*Boc-Ala-Phe-Arg-MCA | 1 |
| | *ⁱ*Boc-Ala-Lys-Arg-MCA | 11 |

| | | |
|---|---|---|
| *¹ Information on purchased tryptase. Enzyme reaction conditions. *² In these compounds, the sulfonic acid (Pmcs, pentamethylchromansulfonic acid) formed a salt as a counter ion with a liberated guanidine group when the Pmc- group, a protective group of the guanidine group of arginine, was removed by trifluoroacetic acid treatment. *³ Relative value with the sensitivity of ⁱBoc-Ala-Ala-Arg-MCA defined as 100. | | |

By the screening described above, we were able to obtain ⁱBoc-Abu-Ala-Arg-MCA and ⁱBoc-Lys-Ala-Arg-MCA as tripeptide substrates having higher tryptase responsiveness than that of ⁱBoc-Ala-Ala-Arg-MCA. Also, we were able to confirm that the tryptase substrate ⁱBoc-Lys-Pro-Arg-MCA having a known tripeptide moiety according to non-patent document 15 have tryptase responsiveness slightly less than two times higher than that of ⁱBoc-Ala-Ala-Arg-MCA. In the -Arg-Asn-Arg- sequence of the tryptase substrate described in non-patent document 18, Asn at position P₂ also exhibits slightly more than two times the responsiveness of Ala at position P₂, and further, at position P₃, an amino acid having a positively charged side chain has sensitivity about two times better than that of Ala at position P₃. In light of these, the substrates were considered to have responsiveness approximately 5 times higher than that of the -Ala-Ala-Arg- sequence, albeit according to calculation.

The following findings were obtained as the outcomes of search for suitable amino acid sequences of the tripeptide.

It is well known as specificity that a proteolytic enzyme (protease) differs in the hydrolysis reaction rate of a peptide bond depending on the structure of the side chain atomic group of an amino acid contained in a protein. Tryptase primarily recognizes Arg having a positively charged side chain and hydrolyzes a peptide bond on the carboxyl group side thereof. The position of an amino acid that is primarily recognized by an enzyme is defined as position P₁, and amino acids toward the N terminus therefrom are designated as positions P₂ and P₃ in order. Even such one amino acid may have not small influence on difference in the rate of enzyme reaction. Amino acids in proteins are broadly divided, according to their side chains, into 4 types, acidic amino acids, basic amino acids, hydrophobic amino acids, and neutral hydrophilic amino acids. These amino acids may be typified by Glu, Arg, Phe, and Ser, respectively. Accordingly, Gly, Ala, Val, Phe, Ser, or Pro was introduced as the amino acid at position P₂ while Glu was excluded therefrom. Likewise, each of the typical amino acids was also introduced to position P₃. Lys was also used at position P₃. Since a number of compounds needed to be synthesized, a method for purifying synthetic intermediates was simplified and a safe ⁱBoc group was used for trifluoroacetic acid in the removal of a protective group in the final step. After removal of the Pmc protective group on the side chain of Arg with trifluoroacetic acid, the product formed a salt with the side chain of Arg. Therefore, most of ⁱBoc-tripeptide-MCA compounds were obtained in a favorable powder form.

As a result of studying the experimental results of Table 1, we were able to summarize features of the amino acid sequences of the substrates for tryptase as the following 5 points. (i) It is evident that Gly at position P₂ is not suitable. (ii) Ala with a methyl group having a small side chain is most suitable for position P₃, and a bulky amino acid such as Val or Phe decreased responsiveness. A large side chain may be difficult for subsite S₂ of the enzyme to accommodate. Pro is an amino acid that also often appears at position P₂ in substrates for other proteases. (iii) Non-protein Abu exhibited slightly lower responsiveness than that of Ala. (iv) At position P₃, as in position P₂, a small side chain of an amino acid such as Ala or Abu is acceptable, and a bulky hydrophobic side chain is difficult to bind. (v) On the other hand, positively charged Lys was found to be accommodated at position P₃. Provided that a natural substrate protein for tryptase will be identified in the future in relation to pathology and the amino acid sequence of its site of action will be determined, Lys will be found at position P₃ thereof.

### Example 2

### 2. Synthesis of macromolecular substrate in which Suc-Ala-Ala-Arg-MCA is linked to poorly tryptase-digestible water-soluble polymer

A substrate for measuring tryptase activity linked to poly(L-lysine) as a poorly tryptase-digestible water-soluble polymer was synthesized by the following method.
(1) Fmoc-Arg(Pmc)-MCA (own made, 1.1 mmol) was dissolved in 20% piperidine in DMF (10 mL) and reacted for 90 minutes. DMF was distilled off to obtain free amine in a white solid form. This solid was dissolved in DMF (5 mL). To the solution, Boc-Ala-Ala-OH (own made, 1.2 mmol) was added, followed by condensation by the HBTU/HOBt method. After reaction for 4 hours, Boc-Ala-Ala-Arg(Pmc)-MCA was isolated and purified by silica gel chromatography [yield: 505 mg (62%)].
(2) Boc-Ala-Ala-Arg(Pmc)-MCA (440 mg) was dissolved in TFA (5 mL) and reacted for 10 minutes. TFA was rapidly distilled off, and the residue was crystalized with diethyl ether.
(3) TFA_H-Ala-Ala-Arg(Pmc)-MCA (390 mg) was dissolved in DMF (5 mL). Under ice cooling, NEt₃ (2.2 eq.) and succinic anhydride (1.2 eq.) were added to the solution, and the mixture was reacted overnight at room temperature. Formed Suc-Ala-Ala-Arg(Pmc)-MCA was extracted and isolated, further dissolved in TFA (3 mL), and reacted for 2 hours to remove the Pmc group.
(4) Poly(L-lysine) hydrochloride having a molecular weight of 8,000 or higher (manufactured by Peptide Institute, Inc., Lot. 670515, 165 mg, 1 mmol) was dissolved in water (5 mL). To the solution, Suc-Ala-Ala-Arg-MCA_Pmcs (164 mg, 0.2 mmol) was added, and the hydrochloride was neutralized with NaHCO₃ (21 mg, 0.5 mmol). Further, pH was kept at 8 to 9 by the addition of N-hydroxysuccinimide (HOSu, 23 mg, 0.2 mmol) and 1-ethyl-3-(3-dimethylaminopropyl)-carbodiimide_HCl (EDC, MW 191.7, 96 mg, 0.5 mmol). Since the reaction solution foamed, ethanol (1 mL) was added as an antifoaming agent 5 hours later. Reaction was performed for 2 days.
(5) The reaction solution was concentrated by the addition of acetic acid in a small amount. When ethanol was added to the residual syrup, it was solidified. On the next day, the solvent was removed by decantation, and the residue was dissolved again in distilled water. The solution was concentrated with ethanol as an antifoaming agent, and ethanol was added to the residual syrup to obtain a white solid. 220 mg of Suc-Ala-Ala-Arg-MCA-linked poly(L-lysine) was obtained by vacuum drying.
(6) 3.81 mg of Suc-Ala-Ala-Arg-MCA-linked poly(L-lysine) was dissolved in 50 mL of distilled water. When an absorption spectrum was measured, absorbance at 280 nm was 0.55.
   Also, a substrate for measuring tryptase activity linked to poly(D-lysine) as a poorly tryptase-digestible water-soluble polymer was synthesized by the following method.
(7) Tripeptide-MCA was condensed with poly(D-lysine) by the same procedures as in the steps (1) to (6) except that poly(D-lysine) hydrobromide having a molecular weight of 4,000 to 15,000 (manufactured by Sigma-Aldrich Co. LLC, Lot. SLBZ6409, 42 mg, 0.2 mmol) and Suc-Ala-Ala-Arg-MCA_Pmcs (33 mg, 0.04 mmol) were used in the step (4). 38 mg of the title compound was obtained.
   Dendritic poly(lysine) was considered as a macromolecule suitable for suppressing the access of MCA substrates to blood coagulation system protease such as thrombin trapped by α2 macroglobulin tetramer. Accordingly, dendritic poly(L-lysine) and dendritic poly(D-lysine) were prepared by the following method.
(8) Two molecules of di-Boc-L-lysine were condensed with hexamethylenediamine. After removal of the Boc groups with TFA, four molecules of di-Boc-L-lysine were condensed with four liberated amino groups (second-generation K6B8 wherein K represents the number of L-lysine molecules, and B represents the number of Boc groups). This operation was repeated to expand the dendrite to third-generation K14B16, fourth-generation K30B32, and fifth-generation K62B64 (Chem. Commun., 1999, 2057-2058 (1999)). K14B16, K30B32, and K62B64 were treated with TFA to obtain TFA salts of K14A16 (wherein A represents a free amino group (TFA salt) after deprotection), K30A32, and K62A64 as white powders (Table 2) .
(9) TFA salts of k14A16 (wherein k represents the number of D-lysine molecules), k30A32, and k62A64 were obtained as white powders by the same procedures as above except that di-Boc-D-lysine was used instead of di-Boc-L-lysine in the step (8) (Table 2).

**[Table 2]**

| The numbers of lysine molecules, the numbers of free amino groups, molecular weights as TFA salt, synthesis yields and yield percentages of dendritic poly(L-lysine) and poly(D-lysine) in each generation | | | | |
|---|---|---|---|---|
| Generation | Abbreviation | The number of free amino groups^{*)} | Molecular weight (+ TFA salt) | Synthesis yield and yield percentage |
| 3G | K14A16 | 16 | 1908+1824 | 902 mg(86%) |
| 4G | K30A32 | 32 | 3956+3648 | 762 mg(77%) |
| 5G | K62A64 | 64 | 8052+7296 | 341 mg(75%) |
| 3G | **k**14A16 | 16 | 1908+1824 | 1.85 g(83%) |
| 4G | **k**30A32 | 32 | 3956+3648 | 1.13 g(80%) |
| 5G | **k**62A64 | 64 | 8052+7296 | 962 mg(75%) |

| | | | | |
|---|---|---|---|---|
| *) **k** is an abbreviation of D-lysine. | | | | |

The obtained dendritic poly(lysine) was linked to Suc-Ala-Ala-Arg-MCA by the following method.

(10) The dendritic poly(D-lysine) 5Gk62A64 (316 mg) was dissolved in water (10 mL). To the solution, Suc-Ala-Ala-Arg-MCA_Pmcs (330 mg, 0.4 mmol) was added, and the trifluoroacetate was neutralized with NaHCO₃ (43 mg, 1 mmol). Further, N-hydroxysuccinimide (HOSu, 50 mg, 0.4 mmol) and 1-ethyl-3-(3-dimethylaminopropyl)-carbodiimide_HCl (EDC, 200 mg, 1 mmol) were added thereto, followed by reaction as described in the step (4). 343 mg of 5Gk62A64-Suc-Ala-Ala-Arg-MCA acetate was obtained.

### Example 3

### 3. Activity measurement of tryptase in serum using substrate of present invention

Suc-Ala-Ala-Arg-MCA-linked poly(L-lysine) prepared in Example 2 was used to test whether to be able to specifically measure tryptase activity in serum.

(1) 20.0 mg of Suc-Ala-Ala-Arg-MCA-linked poly(L-lysine) was dissolved in 50 mL of 50 mM Tris-HCl (pH 8.0).
(2) 2 µL of commercially available human serum (manufactured by BioWest) was mixed with 2 µL of PBS(-) (pH 7.4) (control) or 2 µL of 10 µM nafamostat (tryptase inhibitor, manufactured by Tokyo Chemical Industry Co., Ltd.) or 2 µL of 10 U/mL hirudin (thrombin inhibitor, manufactured by Sigma-Aldrich Co., LLC). After a lapse of 10 minutes at room temperature, each mixed solution was added to 100 µL of the solution prepared in the step (1). Immediately thereafter, enzyme activity was measured.

The results are shown in the following Table 3.

**[Table 3]**

| Comparison of human serum enzyme activity by addition of various inhibitors | |
|---|---|
| Reaction solution | Enzyme activity *¹ |
| Serum + PBS (-) | 100 |
| Serum + nafamostat | 0 |
| Serum + hirudin | 83 |

| | |
|---|---|
| *¹ Relative value with the enzyme activity of serum + PBS(-) defined as 100 | |

As seen from Table 3, the enzyme activity in serum detected with the macromolecular substrate of the present invention was completely inhibited by the tryptase inhibitor nafamostat, but not by the thrombin inhibitor hirudin. These results indicated that the enzyme activity in serum detected with the macromolecular substrate of the present invention was ascribable to tryptase and the tryptase activity can be specifically measured even in a serum sample containing a blood coagulation system enzyme such as thrombin in a state trapped by α2 macroglobulin tetramer.

### Industrial Applicability

The present invention enables tryptase activity in a blood sample to be directly measured without the pretreatment, such as purification or concentration, of the blood sample. Hence, mast cell activation syndrome, which has been determined so far only from the abundance in blood of a tryptase protein-related antigen, can be determined from tryptase activity which more accurately reflects the disease state. Therefore, the present invention has high industrial applicability in the medical field.

## Claims

1. A substrate for measuring tryptase activity, comprising a tripeptide C-terminally linked through a peptide bond to a dye label (tripeptide-X), selected from the following formulas (1) to (3):
(1)
Lys-Ala-Arg-X,
(2)
Ala-Ala-Arg-X,
and
(3)
Abu-Ala-Arg-X
(wherein X represents a dye label whose fluorescence characteristics or color development characteristics change upon the cleavage of the peptide bond with Arg, and Abu represents 2-aminobutyric acid).

2. The substrate for measuring tryptase activity according to claim 1, wherein the substrate for measuring tryptase activity is for directly measuring tryptase activity in a blood sample.

3. The substrate for measuring tryptase activity according to claim 1 or 2, wherein the tripeptide-X is N-terminally linked to a poorly tryptase-digestible water-soluble polymer having a molecular weight of 5,000 or higher.

4. The substrate for measuring tryptase activity according to claim 3, wherein the poorly tryptase-digestible water-soluble polymer is a polyamino acid.

5. The substrate for measuring tryptase activity according to claim 4, wherein the polyamino acid is selected from poly(L-lysine), dendritic poly(L-lysine), poly(D-lysine), dendritic poly(D-lysine), poly(L-ornithine) and poly(D-ornithine).

6. The substrate for measuring tryptase activity according to any one of claims 1 to 5, wherein the dye label is a fluorescent dye label selected from an MCA group, an ANS group, a CMCA group, an FMCA group, an AMP group, a rhodamine 110 group, a rhodamine 110 monoamide group, a rhodamine 6G group and a rhodamine B group.

7. A method for measuring tryptase activity in a blood sample, comprising the following steps (A) and (B):
(A) contacting the substrate for measuring tryptase activity according to any one of claims 1 to 6 with the blood sample; and
(B) calculating a degree of the tryptase activity in the blood sample by measuring an amount of change in fluorescence characteristics or color development characteristics of a dye label after the step (A).

8. The method according to claim 7, wherein the blood sample is serum.

9. A kit for measuring tryptase activity in a blood sample, comprising the substrate for measuring tryptase activity according to any one of claims 1 to 6.

10. The kit according to claim 9, wherein the blood sample is serum.
